# EUROPÄISCHE PATENTANMELDUNG

(11) **EP 0 842 640 A1**
(43) Veröffentlichungstag der Anmeldung: **20.05.1998**
(21) Anmeldenummer: 96810784.7
(22) Anmeldetag: 13.11.1996
(51) Int. Cl.: A61B 17/39

(54) **Herzkatheter mit Elektrode auf aufspannbarer Einrichtung**

(71) Anmelder: Sulzer Osypka GmbH, 79639 Grenzach-Wyhlen (DE)
(72) Erfinder: Osswald, Stefan, CH-4054 Basel (CH); Eberhart, Andreas, D-79539 Lörrach (DE); Baumgartner, Daniel, D-79639 Grenzach-Wyhlen (DE)
(74) Vertreter: Heubeck, Bernhard

(57) **Zusammenfassung**

Der Herzkatheter weist mindestens einen Elektrodenpol (3) auf, der auf einer aufspannbaren Einrichtung (1) an dem distalen Ende eines Katheterschafts (2) angeordnet ist. Die Einrichtung umfasst mindestens zwei aus einem elastischen Material gefertigte Streben (6), die den Konturen eines Teilraums des Herzes, insbesondere eines Vorhofs folgend aufspannbar sind. Es sind Mittel (4, 7) vorgesehen sind, mit welchen der Elektrodenpol (3) entlang einer Strebe (6) verschiebbar ist.

## Beschreibung

Die Erfindung betrifft einen Herzkatheter mit Elektrode auf aufspannbarer Einrichtung gemäss Oberbegriff von Anspruch 1.

Aus der Patentschrift US 5 263 493 ist ein derartiger Herzkatheter bekannt. Im Bereich des distalen Endes dieses Katheters ist eine aufspannbare, zwei Streben umfassende Einrichtung angeordnet; auf den Streben befindet sich eine Vielzahl von einzeln ansteuerbaren Elektrodenpolen. Bei nicht aufgespanntem Zustand der Elektrodeneinrichtung kann der Katheter über ein Blutgefäss in einen Teilraum des Herzes eingeschoben werden. Im Herz wird die Elektrodeneinrichtung aufgespannt, so dass die Elektrodenpole in Kontakt mit der inneren Oberfläche des Teilraums treten. Mit den Elektrodenpolen lassen sich Herzpotentiale abtasten (endokartiales "mapping"). Sie lassen sich auch für das Durchführen von Ablationen in einem Vorhof des Herzes verwenden.

Aufgabe der Erfindung ist es, einen Herzkatheter zu schaffen, der sich besonders gut für die Durchführung von Ablationen eignet. Diese Aufgabe wird durch den in Anspruch 1 definierten Katheter gelöst.

Der Herzkatheter weist mindestens einen Elektrodenpol auf, der auf einer aufspannbaren Einrichtung an dem distalen Ende eines Katheterschafts angeordnet ist. Die Einrichtung umfasst mindestens zwei aus einem elastischen Material gefertigte Streben, die den Konturen eines Teilraums des Herzes, insbesondere eines Vorhofs folgend aufspannbar sind. Es sind Mittel vorgesehen sind, mit welchen der Elektrodenpol entlang einer Strebe verschiebbar ist. Mit dein bewegbaren Elektrodenpol gelingt es, auf der inneren Oberfläche des Herzes zusammenhängende Ablationslinien (Linienläsionen) zu ziehen. Die abhängigen Ansprüche 2 bis 10 betreffen vorteilhafte Ausbildungsformen des erfindungsgemässen Katheters.

Nachfolgend wird die Erfindung anhand eines in den Zeichnungen dargestellten Ausführungsbeispiels erläutert. Es zeigen:
- Fig. 1: die am distalen Ende eines Herzkatheters angeordnete Elektrodeneinrichtung in einem aufgespannten Zustand,
- Fig. 2: die Elektrodeneinrichtung in einem gestreckten Zustand, wobei lediglich ein Bereich des Kopfteils dargestellt ist,
- Fig. 3: den auf einer Strebe bewegbaren Elektrodenpol,
- Fig. 4: einen Schnitt durch den Elektrodenpol und
- Fig. 5: Gelenkverbindungen der Streben am distalen Ende des Katheterschafts.

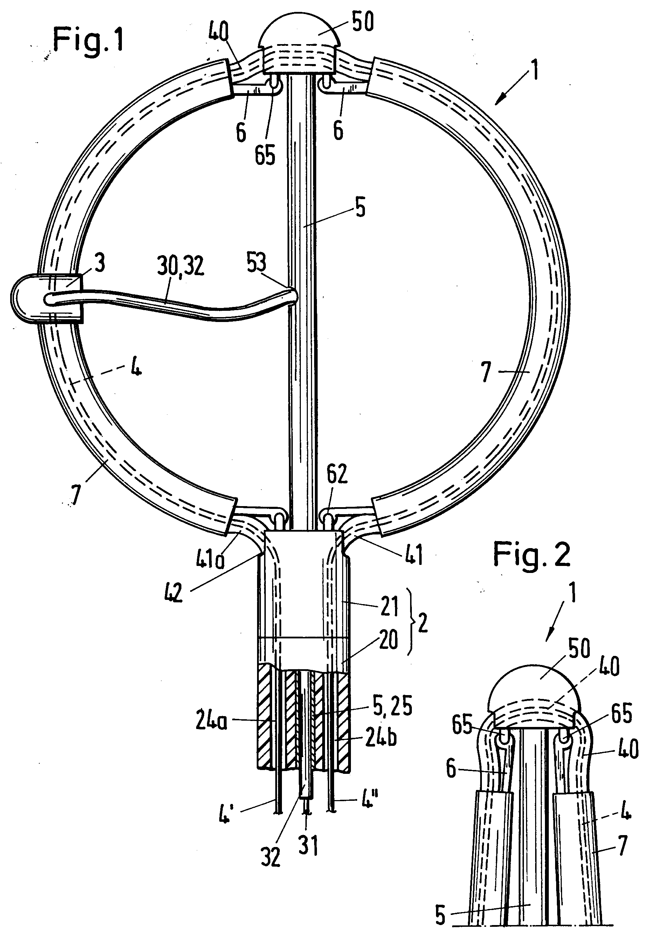

Das in Fig.1 dargestellte distale Ende eines erfindungsgemässen Herzkatheters umfasst folgende Komponenten: eine aufgespannte Elektrodeneinrichtung 1 am distalen Ende eines Katheterschafts 2; einen verschiebbaren Elektrodenpol 3, der mit einem schlaufenartigen Zugfaden 4 bewegbar ist; ein Führungsrohr 5; ferner Streben 6 mit Führungen 7 in Form von Profilschläuchen.

Der Katheterschaft 2 besteht aus einem mehrlumigen Schlauch 20 und einer Schafthülse 21. Die Führungen 7 für den Zugfaden 4, der mit dem Elektrodenpol 3 verbunden ist, sind entlang zweier einander gegenüberliegender Streben 6 angeordnet. Die beiden Fortsetzungen 4' und 4'' des schlaufenartigen Zugfadens 4 werden durch zwei Lumen 24a und 24b des Katheterschafts 2 zu einem nicht dargestellten proximalen Ende des Katheters geführt.

Der Katheterschaft 2 enthält mindestens in seinem distalen Endbereich ein Lumen 25 für das Führungsrohr 5, das in dem Lumen 25 verschiebbar ist und teilweise aus ihm herausragt. Die Streben 6 sind zwischen einer Kopfhülse 50 am distalen Ende des Führungsrohrs 5 und dem distalen Ende des Katheterschafts 2 angeordnet. Sie sind über Gelenke 65 mit der Kopfhülse 50 und über Gelenke 62 mit der Schafthülse 21 verbunden. Die Einrichtung 1 kann durch Bewegen des Führungsrohrs 5 in das Lumen 25 hinein oder aus ihm heraus von einem gestreckten (Fig.2) in einem korbartigen Zustand (Fig.1) oder umgekehrt geändert werden.

Im Kopfbereich der Einrichtung 1 wird der Zugfaden 4 durch einen die beiden Führungen 7 verbindenden Schlauch 40 geführt, der in die Kopfhülse 50 eingelegt ist. Entsprechende Übergangsstücke an den anderen Enden der Führungen 7 werden durch Schläuche 41a und 41b gebildet, die in die Lumen 24a beziehungsweise 24b einmünden.

Im Führungsrohr 5 ist koaxial eine elektrische, mit einem Schutzschlauch 32 ummantelte Leitung 31 angeordnet, die zwischen einer Öffnung 53 im freiliegenden Teil des Führungsrohrs 5 und dem Elektrodenpol 3 eine flexible Verbindung 30 herstellt.

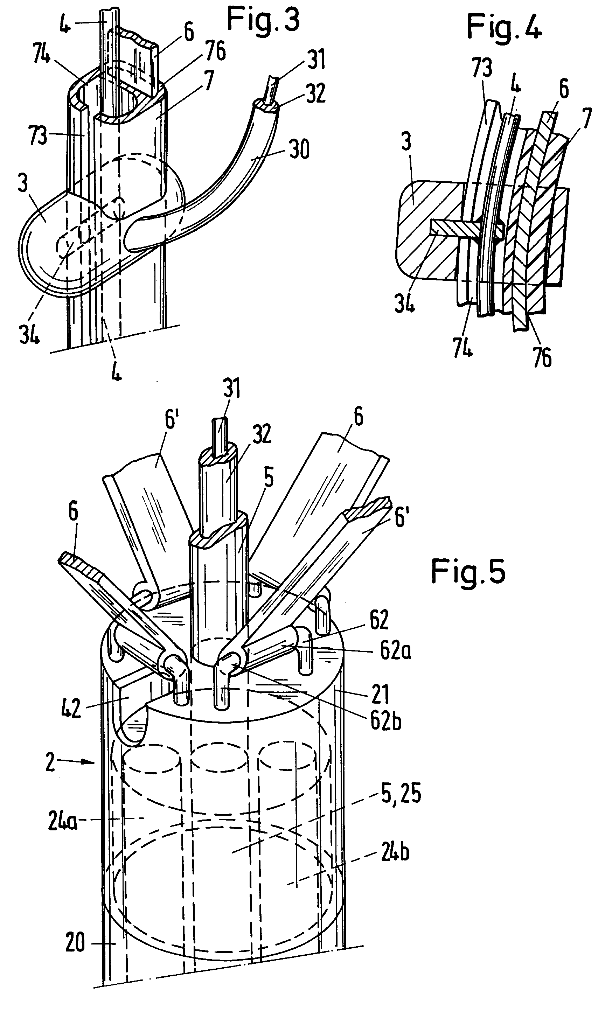

Der Zugdraht 4 ist in den Bereichen der Streben 6 jeweils in einem Lumen 74 der als Profilschlauch ausgebildeten Führung 7 eingelegt: siehe Fig.3. Der Profilschlauch 7 weist ein zweites Lumen 76 auf, durch das die Strebe 6 führt und damit eine Befestigung der Führung 7 auf der zugeordneten Strebe 6 herstellt. Die elektrische Verbindung 30 ist an einer Seite des Elektrodenpols 3 angebracht. Wie aus der Schnittzeichnung in Fig.4 hervorgeht, ist der Elektrodenpol 3 über einen Mitnehmer 34 mit dem Zugfaden 4 verbunden. Eine Nut 73 ist für den Durchtritt des Mitnehmers 34 im Profilschlauch 7 vorgesehen.

Wie Fig.5 zeigt, sind vier Streben 6, 6' vorgesehen, die zwei Paare von jeweils diametral angeordneten Streben 6 beziehungsweise 6' bilden. Das Paar mit den Streben 6 trägt die Mittel, nämlich die Führungen 7 und den Zugfaden 4, mit welchen der Elektrodenpol 3 entlang einer der beiden Streben 6 verschiebbar ist.

Die Streben 6, 6' sind mittels Hülsen 62a und Bolzen 62b der Gelenke 62 an der Deckplatte der Schafthülse 21 befestigt. Entsprechende Befestigungen mit Gelenken 65 sind an einer Gelenkplatte an der Basis der Kopfhülse 50 vorgesehen. Die Befestigungsart mit Gelenken 62, 65 ermöglicht ein orthogonales Aufspannen der Streben 6, 6' gegenüber dem Katheterschaft 2. Das orthogonale Aufspannen ergibt eine Kugelform der Einrichtung 1 (vgl. Fig.1). Durch die Kugelform wird der zu behandelnde Vorhof optimal ausgefüllt sowie aufgespannt.

Nicht eingezeichnet sind in Fig.5 die Schlauchstücke 41a und 41b für den Zugfaden 4 (siehe Fig.1) und die Führungen 7. Das eingesetzte Schlauchstück 41a würde eine Verbindung vom Lumen 24a durch die Öffnung 42 zu dem Lumen 74 (siehe Fig.3) der Führung 7 herstellen.

Die Streben 6, 6' sind im wesentlichen in Form von biegbaren, geraden Streifen ausgebildet, wobei die lange Seite des rechteckigen Strebenquerschnitts in einer zur Katheterschaftachse senkrechten sowie tangentialen Richtung liegt. Die Streben 6, 6' sind mit Vorteil aus einer metallischen Legierung gefertigt (beispielsweise aus "Elgiloy"). Der Strebenquerschnitt kann auch rund, beispielsweise kreisförmig oder elliptisch sein.

Die Anzahl der Streben 6 kann auch grösser oder kleiner als vier sein, muss aber mindestens zwei betragen. Es können auch mehr als ein Elektrodenpol 3 - auf der gleichen oder auf verschiedenen Streben angeordnet - vorgesehen sein.

Der Elektrodenpol 3 gemäss Fig.3 ist so ausgebildet, dass er auf der Oberfläche des Profilschlauchs 3 geführt wird. Für die Führung des Elektrodenpols 3 kann auch eine Schiene (nicht dargestellt) verwendet werden.

Die elektrische Verbindung 30 kann auch entlang der Streben 6 hergestellt werden, indem man beispielsweise den Zugfaden so ausbildet, dass er die elektrische Leitung 31 enthält. In diesem Fall kann das Lumen des Führungsrohrs 5 für die Aufnahme eines Führungsdrahts genutzt werden, mit dessen Hilfe der Herzkatheter sich durch die Blutgefässe zum Herz und in dieses hinein schieben lässt.

Wird für die Einführung des Katheters in das Herz kein Führungsdraht verwendet, so ist es vorteilhaft, wenn am distalen Ende der Kopfhülse 50 eine flexible, 2 bis 3 cm lange Spitze (nicht dargestellt) vorgesehen ist. Diese Spitze erleichtert das Einschieben des Katheters.

Der Elektrodenpol 3 ist über die Verbindung 30 an eine für die Durchführung einer Ablation geeignete, geregelte und ausserhalb des Körpers des Patienten angeordnete Energiequelle (Hochfrequenz HF) anschliessbar. Zu diesem Zweck ist der Pol 3 mit Vorteil mit einem Thermosensor (Thermoelement, Thermistor) ausgestattet. Die Verbindung 30 enthält neben der elektrischen Leitung 31 zusätzlich zwei Anschlussleitungen zu dem Thermosensor.

Es kann auch mindestens ein zweites Mittel zur Durchführung von diagnostischen und/oder therapeutischen Herzeingriffen vorgesehen sein, welches durch ein separates Lumen des Katheterschafts (nicht dargestellt) in das Herz einführbar ist. Dabei kann die Verwendung einer Strebe als Führungshilfe vorteilhaft sein. Ein Beispiel für das genannte zweite Mittel ist ein Elektrodenkatheter, mit dem sich Herzpotentiale abtasten lassen.

## Patentansprüche

1. Herzkatheter mit Elektrode auf aufspannbarer Einrichtung (1) an dem distalen Ende eines Katheterschafts (2), welche mindestens zwei aus einem elastischen Material gefertigte Streben (6) umfasst, die den Konturen eines Teilraums des Herzes, insbesondere eines Vorhofs folgend aufspannbar sind,
dadurch gekennzeichnet, dass Mittel (4, 7) vorgesehen sind, mit welchen mindestens ein Elektrodenpol (3) entlang einer Strebe (6) verschiebbar ist.

2. Herzkatheter nach Anspruch 1, dadurch gekennzeichnet, dass entlang zweier Streben (6) Führungen (7) für einen schlaufenartigen Zugfaden (4) angeordnet sind, der zum Verschieben des Elektrodenpols (3) mit diesem verbunden ist und dessen beiden Fortsetzungen (4', 4'') durch zwei Lumen (24a, 24b) des Katheterschafts (2) zum proximalen Ende des Katheters geführt sind.

3. Herzkatheter nach Anspruch 1 oder 2, dadurch gekennzeichnet, dass der Katheterschaft (2) mindestens in seinem distalen Endbereich ein Lumen (25) für ein Führungsrohr (5) enthält, dass das Führungsrohr (5) in dem Lumen (25) verschiebbar ist und teilweise aus ihm herausragt, dass die Streben (6) zwischen einer Kopfhülse (50) am distalen Ende des Führungsrohrs und dem distalen Ende des Katheterschafts (2) angeordnet sind und dass insbesondere die Streben über Gelenke (65, 62) mit der Kopfhülse sowie einer Schafthülse (21) verbunden sind, wobei die Einrichtung (1) zwischen einem gestreckten und einem korbartigen Zustand durch Bewegen des Führungsrohrs veränderbar ist.

4. Herzkatheter nach einem der Ansprüche 1 bis 3, dadurch gekennzeichnet, dass im Führungsrohr (5) koaxial mindestens eine elektrische, mit einem Schutzschlauch (32) ummantelte Leitung (31, 30) für den Elektrodenpol angeordnet ist und dass diese Leitung zwischen einer Öffnung (53) im freiliegenden Teil des Führungsrohrs und dein Elektrodenpol (3) eine flexible Verbindung (30) herstellt.

5. Herzkatheter nach einem der Ansprüche 2 bis 4, dadurch gekennzeichnet, dass der Zugdraht (4) in den Bereichen der Streben (6) durch Führungsmittel (7, 74) geführt ist.

6. Herzkatheter nach einem der Ansprüche 1 bis 5, dadurch gekennzeichnet, dass vier Streben (6, 6') vorgesehen sind, die zwei Paare von jeweils diametral angeordneten Streben bilden.

7. Herzkatheter nach Anspruch 6, dadurch gekennzeichnet, dass eines der Paare als Träger für jene Mittel (4, 7) vorgesehen ist, mit welchen der Elektrodenpol (3) entlang einer der beiden Streben (6) des Paares verschiebbar ist.

8. Herzkatheter nach einem der Ansprüche 1 bis 7, dadurch gekennzeichnet, dass die Streben (6) aus einer metallischen Legierung gefertigt sind und im wesentlichen in Form von biegbaren, geraden Streifen ausgebildet sind, wobei die lange Seite des rechteckigen Strebenquerschnitts in einer zur Achse des Katheterschafts (2) senkrechten sowie tangentialen Richtung liegt.

9. Herzkatheter nach einem der Ansprüche 1 bis 7, dadurch gekennzeichnet, dass der Elektrodenpol (3) mit einer für die Durchführung einer Ablation geeigneten, geregelten und ausserhalb des Körpers des Patienten angeordneten Energiequelle verbindbar ist.

10. Herzkatheter nach Anspruch 9, dadurch gekennzeichnet, dass mindestens ein zweites Mittel zur Durchführung von diagnostischen und/oder therapeutischen Herzeingriffen vorgesehen ist, welches durch ein separates Lumen des Katheterschafts in das Herz einführbar und dort insbesondere unter Verwendung einer Strebe (6) als Führungshilfe bewegbarbar ist, wobei das zweite Mittel beispielsweise ein Elektrodenkatheter zum Abtasten von Herzpotentialen ist.
